# EUROPÄISCHE PATENTANMELDUNG

(11) **EP 4 086 649 A1**
(43) Veröffentlichungstag der Anmeldung: **09.11.2022**
(21) Anmeldenummer: 21172537.9
(22) Anmeldetag: 06.05.2021
(51) Int. Cl.: G01R 33/56, G01R 33/561, G06N 3/02

(54) **ADAPTIVE REKONSTRUKTION VON MR-DATEN**

(71) Anmelder: Siemens Healthcare GmbH, 91052 Erlangen (DE)
(72) Erfinder: Paul, Dominik, 91088 Bubenreuth (DE); Bauer, Simon, 96148 Baunach (DE); Dispenza, Nadine, 91052 Erlangen (DE); Nickel, Marcel Dominik, 91074 Herzogenaurach (DE)

(57) **Zusammenfassung**

Die Erfindung betrifft eine adaptive Rekonstruktion von MR-Daten umfassend akquirierte MR-Daten eines Kernbereiches (50) umfassend Kernsegmente (51, 52, 53, 54, 55) und simulierte MR-Daten eines Peripheriebereiches (42) gemäß den folgenden Verfahrensschritten:
- Ermittlung eines Peripheriesignals basierend auf den MR-Daten des Peripheriebereiches (42),
- Bestimmung eines Skalierungsfaktors für jedes Kernsegment (51, 52, 53, 54, 55) unter Berücksichtigung des Peripheriesignals und einer mittleren Signalintensität der MR-Daten für das jeweilige Kernsegment (51, 52, 53, 54, 55),
- Skalierung der MR-Daten des Kernbereiches (50) unter Berücksichtigung der MR-Daten jedes Kernsegmentes (51, 52, 53, 54, 55) und dem des jeweiligen Kernsegmentes (51, 52, 53, 54, 55) entsprechenden Skalierungsfaktors,
- Erzeugung gefilterter MR-Daten durch Kombination der skalierten MR-Daten des Kernbereiches (50) mit den MR-Daten des Peripheriebereiches (42),
- Rekonstruktion von Bilddaten aus den gefilterten MR-Daten.

## Beschreibung

Die Erfindung betrifft ein Verfahren, eine Rekonstruktionseinheit, ein Magnetresonanzgerät, ein Computerprogrammprodukt sowie einen elektronisch lesbaren Datenträger zu einer adaptiven Rekonstruktion von MR-Daten.

In einem Magnetresonanzgerät wird üblicherweise der zu untersuchende Körper eines Untersuchungsobjektes, insbesondere eines Patienten, mit Hilfe eines Hauptmagneten einem relativ hohen Hauptmagnetfeld, beispielsweise von 1,5 oder 3 oder 7 Tesla, ausgesetzt. Zusätzlich werden mit Hilfe einer Gradientenspuleneinheit Gradientenpulse ausgespielt. Über eine Hochfrequenzantenneneinheit werden dann mittels geeigneter Antenneneinrichtungen hochfrequente Hochfrequenz-Pulse, beispielsweise Anregungspulse, ausgesendet, was dazu führt, dass die Kernspins bestimmter, durch diese Hochfrequenz-Pulse (HF-Pulse) resonant angeregter Atome um einen definierten Flipwinkel gegenüber den Magnetfeldlinien des Hauptmagnetfelds verkippt werden. Bei der Relaxation der Kernspins werden Hochfrequenz-Signale, so genannte Magnetresonanz-Signale (MR-Signale), abgestrahlt, die mittels geeigneter Hochfrequenzantennen empfangen und dann weiterverarbeitet werden. Aus den so akquirierten Rohdaten können schließlich die gewünschten Bilddaten rekonstruiert werden.

Für eine bestimmte Messung ist daher eine bestimmte Magnetresonanz-Steuerungssequenz (MR-Steuerungssequenz), auch Pulssequenz genannt, auszusenden, welche aus einer Folge von Hochfrequenz-Pulsen, beispielsweise Anregungspulsen und Refokussierungspulsen, sowie passend dazu koordiniert auszusendenden Gradientenpulsen in verschiedenen Gradientenachsen entlang verschiedener Raumrichtungen besteht. Zeitlich passend hierzu werden Auslesefenster gesetzt, welche die Zeiträume vorgeben, in denen die induzierten MR-Signale erfasst werden.

Der Datenraum, in dem die MR-Signale vorliegen, wird als k-Raum bezeichnet. Die MR-Signale werden digitalisiert und als komplexe Zahlenwerte in einer k-Raum-Matrix entlang von k-Raum-Zeilen in Richtung der Frequenzkodierung abgelegt. Die komplexen Zahlenwerte im k-Raum werden als Rohdaten, insbesondere als MR-Daten, bezeichnet. Aus den Rohdaten können beispielsweise mittels einer Fourier-Transformation zugehörige Bilddaten rekonstruiert werden.

Die Belegung des k-Raumes mit komplexen Zahlenwerten beeinflusst die Bilddaten. Für eine korrekte Widergabe der Anatomie des Untersuchungsobjektes erfüllen die MR-Daten typischerweise das Nyquist-Kriterium. Zusätzlich bestimmt der k-Raum, insbesondere die k-Raum-Matrix, die Auflösung der Bilddaten, also die Größe der Pixel der Bilddaten. Um die Auflösung der Bilddaten zu erhöhen, muss der Wertebereich des k-Raumes vergrößert werden. Je größer die Auflösung der Bilddaten, desto geringer ist die Größe der Pixel der Bilddaten. Die MR-Daten im Zentrum des k-Raums bestimmen den Kontrast der Bilddaten. Das Zentrum des k-Raums ist typischerweise mit MR-Daten belegt.

Die Dauer einer Untersuchung eines Untersuchungsobjektes korreliert typischerweise mit der Menge der aufgenommenen MR-Daten, also der Menge der Rohdaten. Je länger eine Untersuchung dauert, desto unbequemer, fehleranfälliger und kostenintensiver ist eine Untersuchung. Folglich gibt es in der MR-Bildgebung zahlreiche Ansätze zur Reduzierung der aufzunehmenden MR-Daten. Ein Verfahren sieht eine Aufnahme von MR-Daten in einem Kernbereich des k-Raumes vor, wohingegen ein Peripheriebereiches des k-Raumes mittels "Zero-Filling" und/oder simulierten MR-Daten befüllt wird. "Zero-Filling" und/oder eine Simulation von MR-Daten erfordern keine Aufnahme von MR-Daten und verlängern die Untersuchungsdauer typischerweise nicht. Der Wertebereich des k-Raumes kann jedoch mittels "Zero-Filling" und/oder einer Simulation von MR-Daten um den Peripheriebereich vergrößert werden, was die Auslösung der zu rekonstruierenden Bilddaten erhöhen kann. Der Kernbereich des k-Raumes umfasst dabei ein Volumen im k-Raum umgebend das Zentrum des k-Raumes. Der Peripheriebereich des k-Raumes schließt typischerweise an den Kernbereich an und/oder umgibt diesen zumindest teilweise.

Der Erfindung liegt die Aufgabe zugrunde, ein besonders robustes Verfahren zu einer adaptiven Rekonstruktion von MR-Daten umfassend simuliertes Signal im Peripheriebereich des k-Raumes anzugeben. Die Aufgabe wird durch die Merkmale der unabhängigen Ansprüche gelöst. Vorteilhafte Ausgestaltungen sind in den Unteransprüchen beschrieben.

Das erfindungsgemäße Verfahren sieht eine adaptive Rekonstruktion von MR-Daten vor. Das Verfahren umfasst eine Bereitstellung von MR-Daten umfassend MR-Daten eines Kernbereiches eines k-Raumes und MR-Daten eines Peripheriebereiches des k-Raumes. Die MR-Daten des Peripheriebereiches umfassen simuliertes Signal ungleich Null. Der Kernbereich des k-Raumes umfasst zumindest drei Kernsegmente, wobei jedes Kernsegment der zumindest drei Kernsegmente zueinander parallele k-Raum-Zeilen umfasst und die Kernsegmente derart zueinander angeordnet sind, dass die von ihnen umfassten parallelen k-Raum-Zeilen zueinander parallel sind. Die MR-Daten des Kernbereiches sind durch eine Akquisition umfassend ein mehrfaches zeitsequentielles Durchführen der folgenden Schritte, um jeweils die MR-Daten eines Echozuges zu erfassen, gekennzeichnet:
- Schalten eines Anregungspulses,
- Schalten von mehreren Refokussierungspulsen, wobei zwischen zwei aufeinanderfolgenden Refokussierungspulsen je ein Auslesen einer k-Raum-Zeile, insbesondere einem Echo des Echozuges, zugeordnet einem der zumindest drei Kernsegmente erfolgt.

Das erfindungsgemäße Verfahren umfasst zudem die folgenden Verfahrensschritte:
- Ermittlung eines Peripheriesignals basierend auf den MR-Daten des Peripheriebereiches,
- Bestimmung eines Skalierungsfaktors für jedes Kernsegment unter Berücksichtigung des Peripheriesignals und einer mittleren Signalintensität der MR-Daten für das jeweilige Kernsegment,
- Skalierung der MR-Daten des Kernbereiches unter Berücksichtigung der MR-Daten jedes Kernsegmentes und dem des jeweiligen Kernsegmentes entsprechenden Skalierungsfaktors,
- Erzeugung gefilterter MR-Daten durch Kombination der skalierten MR-Daten des Kernbereiches mit den MR-Daten des Peripheriebereiches,
- Rekonstruktion von Bilddaten aus den gefilterten MR-Daten.

Eine k-Raum-Zeile verläuft typischerweise in Richtung der Frequenzkodierung. Eine k-Raum-Zeile ist typischerweise senkrecht zur Phasenkodierrichtung. Ein Kernsegment kann eine k-Raum-Zeile umfassen. Ein Kernsegment kann zumindest zwei k-Raum-Zeilen, bevorzugt zumindest vier k-Raum-Zeilen, besonders bevorzugt zumindest sieben k-Raum-Zeilen umfassen. Ein Kernsegment umfasst in Richtung der Frequenzkodierung typischerweise einen größeren Wertebereich des k-Raumes als in Phasenkodierrichtung. Die zumindest drei Kernsegmente sind typischerweise durch voneinander verschiedene Wertebereiche für die Phasenkodierrichtung gekennzeichnet.

Die MR-Daten des Peripheriebereiches sind typischerweise durch Simulation, insbesondere mittels eines Simulationsalgorithmus, ermittelt worden. Die Simulation der MR-Daten des Peripheriebereiches erfolgte vorzugsweise unter Berücksichtigung der MR-Daten des Kernbereiches. Das erfindungsgemäße Verfahren kann optional die Simulation der MR-Daten des Peripheriebereiches umfassen. Die Verwendung von simulierten MR-Daten umfassend Signal ungleich Null, also eine Signalintensität aufweisend eine Amplitude größer als Null, kann zur Erhöhung der Auflösung der zu rekonstruierenden Bilddaten und damit zu einer verbesserten Qualität der Bilddaten führen.

Die MR-Daten des Kernbereiches wurden vorzugsweise vorab mittels einer Spinecho (SE)-basierten, insbesondere einer Turbospinecho (TSE)-basierten MR-Steuerungssequenz erfasst. Eine derartige MR-Steuerungssequenz erzeugt typischerweise mehrere Echozüge jeweils induziert durch einen Anregungspuls gefolgt von zumindest zwei aufeinanderfolgenden Refokussierungspulsen. Jeder Refokussierungspuls induziert typischerweise ein Echo, während welchen MR-Daten für eine k-Raum-Zeile aufgenommen werden. Eine derartige MR-Steuerungssequenz sieht typischerweise ein zeitsequentielles Durchführen mehrerer Echozüge vor. Ein Anregungspuls ist typischerweise ein HF-Puls induzierend einen Flipwinkel von 90°. Ein Refokussierungspuls ist typischerweise ein HF-Puls induzierend einen Flipwinkel zwischen 90° und 180°.

Die Ermittlung eines Peripheriesignals umfasst vorzugsweise eine Bestimmung einer mittleren Signalintensität der MR-Daten des Peripheriebereiches. Das Peripheriesignal kann beispielsweise einem Mittelwert und/oder einem Median der MR-Daten des Peripheriebereiches entsprechen. Die mittlere Signalintensität der MR-Daten für jedes Kernsegment kann einen Mittelwert und/oder einen Median der der MR-Daten für jedes Kernsegment umfassen. Die Skalierung der MR-Daten des Kernbereiches kann eine Multiplikation der MR-Daten jedes Kernsegmentes mit dem entsprechenden Skalierungsfaktors umfassen.

Die Kombination der skalierten MR-Daten des Kernbereiches mit den MR-Daten des Peripheriebereiches kann eine Skalierung der MR-Daten des Peripheriebereiches unter Berücksichtigung zumindest eines Skalierungsfaktors für zumindest ein Kernsegment umfassen. Die Kombination der skalierten MR-Daten des Kernbereiches mit den MR-Daten des Peripheriebereiches umfasst vorzugsweise ein Ersetzen der MR-Daten des Kernbereiches durch die skalierten MR-Daten des Kernbereiches unter Beibehaltung der MR-Daten des Peripheriebereiches. Die Rekonstruktion von Bilddaten aus den gefilterten MR-Daten umfasst vorzugsweise eine Fouriertransformation der gefilterten MR-Daten.

Die MR-Daten des Kernbereiches und/oder die MR-Daten des Peripheriebereiches und/oder das Peripheriesignal und/oder die skalierten MR-Daten und/oder die gefilterten MR-Daten liegen typischerweise im Rohdatenraum, insbesondere im k-Raum vor. Die rekonstruierten Bilddaten liegen typischerweise im Bildraum vor.

Dieses Verfahren ermöglicht demnach eine adaptive Rekonstruktion abhängig von MR-Daten unter Berücksichtigung verschiedener Signalintensitäten im Kernbereich und den simulierten MR-Daten des Peripheriebereiches. Bedingt durch eine gewählte MR-Steuerungssequenz weisen die Kernsegmente typischerweise unterschiedliche mittlere Signalintensitäten auf, welche sich wiederum von dem Peripheriesignal unterscheiden. Die Skalierung ermöglicht eine Anpassung der Signalintensitäten im Kernbereich an das Peripheriesignal und/oder eine Angleichung der mittleren Signalintensitäten der Kernsegmente zueinander. Hierdurch können durch die MR-Steuerungssequenz, also durch die Aufnahmetechnik bedingte Signalvariationen kompensiert werden. Dies reduziert Artefakte beispielsweise in Form von Ringing in den Bilddaten. Eine herkömmliche Filterung der MR-Daten, insbesondere im Übergangsbereich zwischen Kernbereich und Peripheriebereich, ist insbesondere bei simuliertem Signal ungleich Null nicht realisierbar und würde eine Verschlechterung der Qualität der Bilddaten verursachen. Die erfindungsgemäße adaptiven Rekonstruktion ermöglicht demnach eine robuste Reduktion von Artefakten, insbesondere auch bei simuliertem Signal ungleich Null im Peripheriebereich. Zudem erfolgt das Verfahren automatisiert und damit sehr robust.

Eine Ausführungsform des Verfahrens sieht vor, dass die Bestimmung des Skalierungsfaktors derart erfolgt, dass die Differenz zwischen dem Peripheriesignal und der mittleren Signalintensität der skalierten MR-Daten eines dem Peripheriebereiches benachbarten Kernsegment minimiert wird.

Ein dem Peripheriebereich benachbartes Kernsegment ist vorzugsweise dadurch gekennzeichnet, dass eine k-Raum-Zeile von zwei direkt benachbarten k-Raum-Zeilen vom Kernsegment und die andere k-Raum-Zeile der zwei direkt benachbarten k-Raum-Zeilen vom Peripheriebereich umfasst werden. Abhängig von der verwendeten Technik zur Akquisition der MR-Daten der Kernsegmente kann ein dem Peripheriebereich benachbartes Kernsegment eine mittlere Signalintensität aufweisen, welche beispielsweise zumindest 20% und/oder zumindest 50% und/oder zumindest 100% größer ist als das Peripheriesignal.

Je geringer der Unterschied zwischen den skalierten MR-Daten eines dem Peripheriebereich benachbarten Kernsegmentes und dem Peripheriesignal, desto geringer sind Artefakte wie beispielsweise Ringformen. Eine Minimierung der Differenz zwischen dem Peripheriesignal und der mittleren Signalintensität der skalierten MR-Daten eines dem Peripheriebereiches benachbarten Kernsegment im Rahmen der Bestimmung des Skalierungsfaktors ermöglicht demnach eine besonders gute Reduktion von Artefakten.

Herkömmlich umfasst der Peripheriebereich MR-Daten mit einer Signalintensität von Null, was als "Zero-Filling" bezeichnet wird. Die Grenze zwischen dem Kernbereich, welcher MR-Daten ungleich Null aufweist, und dem Peripheriebereich umfassend MR-Daten mit einer Signalintensität von Null wird herkömmlich mittels eines Filters geglättet, um Artefakte wie Ringing zu vermeiden. Diese Filterung versagt, sofern MR-Daten des Peripheriebereiches simuliertes Signal ungleich Null umfassen. Das vorgeschlagene Verfahren ermöglicht demnach eine Lösung für simuliertes Signal ungleich Null.

Eine Ausführungsform des Verfahrens sieht vor, dass die Bestimmung des Skalierungsfaktors unter Berücksichtigung eines zeitlichen Abstandes dt des Auslesens einer dem jeweiligen Kernsegment zugeordneten k-Raum-Zeile zum Anregungspuls erfolgt. Der zeitliche Abstand dt ist typischerweise durch die MR-Steuerungssequenz, insbesondere durch eine vorgegebene Echozeit T_{E} und/oder eine Abfolge der zu befüllenden k-Raum-Zeilen im Rahmen eines Echozuges für jede k-Raum-Zeile gegeben. Folglich kann auch für jedes Kernsegment ein mittlerer zeitlicher Abstand dt bestimmt werden. Ein Kernsegment kann aber auch ausschließlich k-Raum-Zeilen umfassen, die im Rahmen mehrerer Echozüge jeweils mit gleichem zeitlichen Abstand dt befüllt wurden. Bei einer Akquisition von MR-Daten entsprechend eines Echozuges induziert durch einen Anregungspuls gefolgt von mehreren Refokussierungspulsen ist die Intensität eines MR-Signals typischerweise proportional zu exp(- dt / T₂), wobei T₂ eine T₂-Relaxationszeit zumindest eines Teilbereiches des Untersuchungsbereiches und/oder eine mittlere T₂-Relaxationszeit des Untersuchungsbereiches ist. Es wurde erkannt, dass die mittleren Signalintensität der MR-Daten für das jeweilige Kernsegment abhängig dt ist. Basierend auf dt ist die mittlere Signalintensität der MR-Daten für das jeweilige Kernsegment demnach vorzugsweise mathematisch und/oder theoretisch bestimmbar und es kann auf eine experimentelle Ermittlung der mittleren Signalintensität der MR-Daten für die Kernsegmente verzichtet werden.

Die Berücksichtigung von dt bei der Bestimmung des Skalierungsfaktors unter eines zeitlichen Abstandes dt ermöglicht demnach eine besonders genaue und robuste adaptive Rekonstruktion.

Eine Ausführungsform des Verfahrens sieht vor, dass der zeitliche Abstand dt den Skalierungsfaktor S gemäß der Abhängigkeit S ∼ exp(- T₂ / dt) beeinflusst, wobei T₂ eine mittlere T₂-Relaxationszeit zumindest eines Teilbereiches des Untersuchungsbereiches ist. T₂ kann auch die längste T₂-Relaxationszeit von im Untersuchungsbereich auftretenden Gewebe sein. Unter der Annahme, dass die Intensität eines MR-Signals typischerweise proportional zu exp(- dt / T₂) ist, stellt der Skalierungsfaktor gemäß dieser Ausführungsform sicher, dass die skalierten MR-Daten der Kernsegmente eine gleichmäßige Signalintensität aufweisen. Dies erhöht die Qualität der rekonstruierten Bilddaten.

Eine Ausführungsform des Verfahrens sieht vor, dass der zeitliche Abstand dt den Skalierungsfaktor S gemäß der Abhängigkeit S = A* exp(- T₂ / dt) + B beeinflusst, wobei T₂ eine mittlere T₂-Relaxationszeit zumindest eines Teilbereiches des Untersuchungsbereiches ist und A und B Optimierungsparameter im Rahmen der Bestimmung des Skalierungsfaktors sind. Diese Ausführungsform ermöglicht eine individuelle Optimierung hinsichtlich einer Reduktion möglicher Artefakte.

Eine Ausführungsform des Verfahrens sieht vor, dass A und B derart optimiert werden, dass die Differenz zwischen dem Peripheriesignal und der mittleren Signalintensität der MR-Daten von zwei an den Peripheriebereich direkt angrenzenden Kernsegmenten der zumindest drei Kernsegmente minimiert wird.

Ein an den Peripheriebereich direkt angrenzendes Kernsegment ist vorzugsweise dadurch gekennzeichnet, dass eine k-Raum-Zeile von zwei direkt benachbarten k-Raum-Zeilen vom Kernsegment und die andere k-Raum-Zeile der zwei direkt benachbarten k-Raum-Zeilen vom Peripheriebereich umfasst werden. Die zwei an den Peripheriebereich direkt angrenzenden Kernsegmenten der zumindest drei Kernsegmente liegen vorzugsweise außerhalb des Zentrums des k-Raumes. Die zwei an den Peripheriebereich direkt angrenzenden Kernsegmenten der zumindest drei Kernsegmente sind typischerweise durch zumindest ein Kernsegment voneinander getrennt.

Eine Minimierung der Differenz zwischen dem Peripheriesignal und der mittleren Signalintensität der skalierten MR-Daten beider an den Peripheriebereich direkt angrenzenden Kernsegmente im Rahmen der Bestimmung des Skalierungsfaktors ermöglicht demnach eine besonders gute Reduktion von Artefakten. Die Möglichkeit der entsprechenden Wahl der Optimierungsparameter unter Berücksichtigung des zeitlichen Abstandes dt kann besonders robust und schnell erfolgen.

Eine Ausführungsform des Verfahrens sieht vor, dass A und B derart optimiert werden, dass dem Kernsegment umfassend MR-Daten mit dem zeitlichen Abstand dt entsprechend einer definierten Echozeit T_{E} der Skalierungsfaktor gleich eins zugewiesen wird. Die Optimierungsparameter können gemäß dieser Ausführungsform durch A = exp (T₂/ T_{E}) und/oder B = 0 definiert werden. Typischerweise umfasst das zentrale Kernsegment der zumindest drei Kernsegmente MR-Daten, welche mit einem zeitlichen Abstand dt entsprechend der Echozeit T_{E} zum Anregungspuls akquiriert werden. Das zentrale Kernsegment umfasst typischerweise das Zentrum des k-Raumes. Die Echozeit TE ist typischerweise ein im Rahmen der verwendeten MR-Steuerungssequenz definierter Parameter, welcher den Kontrast der zu rekonstruierenden Bilddaten beeinflusst und/oder eine Abfolge der zu befüllenden k-Raum-Zeilen beeinflusst. Die mittlere Signalintensität der MR-Daten des zentralen Kernsegmentes korreliert typischerweise mit dem Signal-zu-Rauschen-Verhältnis, weshalb eine Reduktion sich auf die Bildqualität der zu rekonstruierenden Bilddaten negativ auswirkt.

Eine Ausführungsform des Verfahrens sieht vor, dass die MR-Daten des Peripheriebereiches mittels eines neuronalen Netzes simuliertes Signal umfassen. Die Simulation der MR-Daten des Peripheriebereiches erfolgt vorzugsweise unter Verwendung einer trainierten Funktion und/oder unter Berücksichtigung der MR-Daten des Kernbereiches und/oder unter Berücksichtigung der MR-Daten zumindest eines dem Peripheriebereich benachbarten Kernsegmentes. Derartige MR-Daten des Peripheriebereiches sind besonders kompatibel mit dem Untersuchungsbereich und/oder den MR-Daten des Kernbereiches. Die Skalierung der MR-Daten des Kernbereiches in Abhängigkeit derart simulierter MR-Daten des Peripheriebereiches und/oder insbesondere deren entsprechenden Peripheriesignals kann besonders präzise erfolgen, sodass die resultierenden rekonstruierten Bilddaten besonders geringe Artefakte aufweisen.

Eine Ausführungsform des Verfahrens sieht vor, dass der zeitliche Abstand dt des Auslesens aller einem Kernsegment der zumindest drei Kernsegmenten zugeordneten k-Raum-Zeilen zum Anregungspuls des entsprechenden Echozuges gleich ist. Zumindest ein Kernsegment, insbesondere das zentrale Kernsegment, umfasst gemäß dieser Ausführungsform ausschließlich k-Raum-Zeilen, die im Rahmen mehrerer Echozüge jeweils mit gleichem zeitlichen Abstand dt befüllt wurden. Dies ermöglicht eine besonders konstante Signalintensität der MR-Daten in dem jeweiligen Kernsegment.

Eine Ausführungsform des Verfahrens sieht vor, dass der zeitliche Abstand dt des Auslesens von zumindest einem Kernsegment der zumindest drei Kernsegmenten zugeordneten k-Raum-Zeilen zum Anregungspuls des entsprechenden Echozuges voneinander verschieden ist. Entsprechend der Länge des Echozuges und der gewählten Echozeit T_{E} kann es zumindest ein Kernsegment geben, welches mit MR-Daten mit variablem zeitlichen Abstand dt befüllt wird. Dieses Kernsegment liegt typischerweise außerhalb des Zentrums des k-Raumes und/oder ist ein dem Peripheriebereich benachbartes Kernsegment. Eine Skalierung der MR-Daten eines solchen Kernsegmentes ist insbesondere wichtig, da dies einen glatten Übergang zum Peripheriebereich ermöglicht und damit Ringartefakte reduziert.

Des Weiteren geht die Erfindung aus von einer Rekonstruktionseinheit umfassend eine Ermittlungseinheit und eine Skalierungseinheit. Die Rekonstruktionseinheit ist dazu ausgebildet, ein erfindungsgemäßes Verfahren zu einer adaptiven Rekonstruktion von MR-Daten auszuführen.

Dafür weist die Rekonstruktionseinheit typischerweise einen Eingang, eine Ermittlungseinheit, eine Bestimmungseinheit, eine Skalierungseinheit, eine Filterungseinheit und einen Ausgang auf. Über den Eingang können der Rekonstruktionseinheit MR-Daten bereitgestellt werden. Weitere, im Verfahren benötigte Funktionen, Algorithmen oder Parameter können der Rekonstruktionseinheit über den Eingang bereitgestellt werden. Die Ermittlungseinheit ist vorzugsweise zu einer Ermittlung eines Peripheriesignals basierend auf den MR-Daten des Peripheriebereiches ausgebildet. Die Bestimmungseinheit ist vorzugsweise zu einer Bestimmung eines Skalierungsfaktors für jedes Kernsegment unter Berücksichtigung des Peripheriesignals und einer mittleren Signalintensität der MR-Daten für das jeweilige Kernsegment ausgebildet. Die Skalierungseinheit ist vorzugsweise zu einer Skalierung der MR-Daten des Kernbereiches unter Berücksichtigung der MR-Daten jedes Kernsegmentes und dem des jeweiligen Kernsegmentes entsprechenden Skalierungsfaktors ausgebildet. Die Filterungseinheit ist vorzugsweise zu einer Erzeugung gefilterter MR-Daten durch Kombination der skalierten MR-Daten des Kernbereiches mit den MR-Daten des Peripheriebereiches und/oder zu einer Rekonstruktion von Bilddaten aus den gefilterten MR-Daten ausgebildet. Die Bilddaten und/oder weitere Ergebnisse einer Ausführungsform des erfindungsgemäßen Verfahrens können über den Ausgang bereitgestellt werden. Der Eingang, die Ermittlungseinheit, die Bestimmungseinheit, die Skalierungseinheit, die Filterungseinheit und der Ausgang sind typischerweise miteinander verbunden.

Ausführungsformen der erfindungsgemäßen Rekonstruktionseinheit sind analog zu den Ausführungsformen des erfindungsgemäßen Verfahrens ausgebildet. Die Rekonstruktionseinheit kann weitere Steuerungskomponenten aufweisen, welche zum Ausführen eines erfindungsgemäßen Verfahrens nötig und/oder vorteilhaft sind. Auf einer Speichereinheit der Rekonstruktionseinheit können Computerprogramme und weitere Software gespeichert sein, mittels derer die Prozessoreinheit der Rekonstruktionseinheit einen Verfahrensablauf eines erfindungsgemäßen Verfahrens automatisch steuert und/oder ausführt.

Ein erfindungsgemäßes Computerprogrammprodukt ist direkt in einer Speichereinheit einer programmierbaren Rekonstruktionseinheit ladbar und weist Programmcode-Mittel auf, um ein erfindungsgemäßes Verfahren auszuführen, wenn das Computerprogrammprodukt in der Rekonstruktionseinheit ausgeführt wird. Dadurch kann das erfindungsgemäße Verfahren schnell, identisch wiederholbar und robust ausgeführt werden. Das Computerprogrammprodukt ist so konfiguriert, dass es mittels der Rekonstruktionseinheit die erfindungsgemäßen Verfahrensschritte ausführen kann. Die Rekonstruktionseinheit muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können. Das Computerprogrammprodukt ist beispielsweise auf einem elektronisch lesbaren Medium gespeichert oder auf einem Netzwerk oder Server hinterlegt, von wo es in den Prozessor einer lokalen Rekonstruktionseinheit geladen werden kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in einer Rekonstruktionseinheit ein erfindungsgemäßes Verfahren durchführen. Beispiele für elektronisch lesbare Datenträger sind eine DVD, ein Magnetband oder einen USB-Stick, auf welchem elektronisch lesbare Steuerinformationen, insbesondere Software, gespeichert ist. Wenn diese Steuerinformationen (Software) von dem Datenträger gelesen und in eine Rekonstruktionseinheit gespeichert werden, können alle erfindungsgemäßen Ausführungsformen der vorab beschriebenen Verfahren durchgeführt werden.

Des Weiteren geht die Erfindung aus von einem elektronisch lesbaren Datenträger, auf dem ein Programm hinterlegt ist, das zu einer Ausführung eines Verfahrens zu einer adaptiven Rekonstruktion von MR-Daten, vorgesehen ist.

Des Weiteren geht die Erfindung aus von einem Magnetresonanzgerät mit einer Steuerungseinheit umfassend eine erfindungsgemäße Rekonstruktionseinheit.

Die Steuerungseinheit ist typischerweise zu einer Ansteuerung des Magnetresonanzgerät gemäße einer MR-Steuerungssequenz ausgebildet. Die MR-Steuerungssequenz kann der Steuerungseinheit über einen Eingang bereitgestellt werden.

Die MR-Steuerungssequenz kann eine Akquisition umfassend ein mehrfaches zeitsequentielles Durchführen der folgenden Schritte vorsehen und/oder initiieren, um jeweils die MR-Daten eines Echozuges zu erfassen:
- Schalten eines Anregungspulses,
- Schalten von mehreren Refokussierungspulsen, wobei zwischen zwei aufeinanderfolgenden Refokussierungspulsen je ein Auslesen einer k-Raum-Zeile, insbesondere einem Echo des Echozuges, zugeordnet einem der zumindest drei Kernsegmente erfolgt.

Die Steuerungseinheit und/oder die Rekonstruktionseinheit kann in das Magnetresonanzgerät integriert sein. Die Steuerungseinheit und/oder die Rekonstruktionseinheit kann auch separat von dem Magnetresonanzgerät installiert sein. Die Steuerungseinheit und/oder die Rekonstruktionseinheit kann mit dem Magnetresonanzgerät verbunden sein.

Das Magnetresonanzgerät kann weitere Steuerungskomponenten aufweisen, welche zum Ausführen eines erfindungsgemäßen Verfahrens nötig und/oder vorteilhaft sind. Auch kann das Magnetresonanzgerät dazu ausgebildet sein, Steuerungssignale zu senden und/oder Steuerungssignale zu empfangen und/oder zu verarbeiten, um ein erfindungsgemäßes Verfahren auszuführen. Auf einer Speichereinheit der Steuerungseinheit können Computerprogramme und weitere Software gespeichert sein, mittels derer die Prozessoreinheit der Steuerungseinheit einen Verfahrensablauf eines erfindungsgemäßen Verfahrens automatisch steuert und/oder ausführt.

Die Vorteile der erfindungsgemäßen Rekonstruktionseinheit, des erfindungsgemäßen Magnetresonanzgeräts, des erfindungsgemäßen Computerprogrammprodukts und des erfindungsgemäßen elektronisch lesbaren Datenträgers entsprechen im Wesentlichen den Vorteilen des erfindungsgemäßen Verfahrens zu einer adaptiven Rekonstruktion von MR-Daten, welche vorab im Detail ausgeführt sind. Hierbei erwähnte Merkmale, Vorteile oder alternative Ausführungsformen können ebenso auch auf die anderen beanspruchten Gegenstände übertragen werden und umgekehrt.

Weitere Vorteile, Merkmale und Einzelheiten der Erfindung ergeben sich aus den im Folgenden beschriebenen Ausführungsbeispielen sowie anhand der Zeichnungen.

Es zeigen:
- Fig. 1: eine erfindungsgemäße Rekonstruktionseinheit in einer schematischen Darstellung,
- Fig. 2: ein erfindungsgemäßes Magnetresonanzgerät in einer schematischen Darstellung,
- Fig. 3: ein Ablaufdiagramm einer ersten Ausführungsform eines erfindungsgemäßen Verfahrens,
- Fig. 4: einen k-Raum in einer schematischen Darstellung,
- Fig. 5: ein Erfassen von MR-Daten des Kernbereiches in einem ersten Echozug in einer schematischen Darstellung,
- Fig. 6: ein Erfassen von MR-Daten des Kernbereiches in einem weiteren Echozug in einer schematischen Darstellung,
- Fig. 7: eine Signalintensität der MR-Daten vor der Skalierung,
- Fig. 8: eine Signalintensität von gefilterten MR-Daten mit einem Skalierungsfaktor gemäß einer ersten Ausführungsform, und
- Fig. 9: eine Signalintensität von gefilterten MR-Daten mit einem Skalierungsfaktor gemäß einer zweiten Ausführungsform.

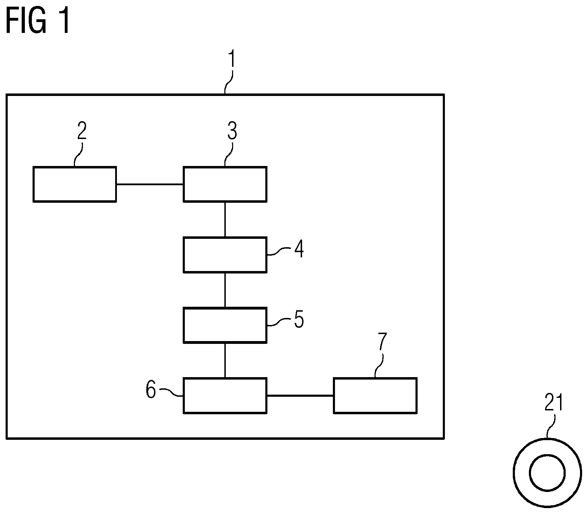

Figur 1 zeigt eine erfindungsgemäße Rekonstruktionseinheit 1 in einer schematischen Darstellung. Die Rekonstruktionseinheit 1 umfasst typischerweise einen Eingang 2, eine Ermittlungseinheit 3, eine Bestimmungseinheit 4, eine Skalierungseinheit 5, eine Filterungseinheit 6 und einen Ausgang 7. Über den Eingang 2 kann die Bereitstellung von MR-Daten erfolgen. Die Ermittlungseinheit 3 ist typischerweise dazu ausgebildet, ein Peripheriesignal basierend auf den MR-Daten des Peripheriebereiches 42 zu ermitteln. Die Ermittlungseinheit 3 kann auch dazu ausgebildet sein, Signal ungleich Null als MR-Daten des Peripheriebereiches 42 zu simulieren. Die Bestimmungseinheit 4 ist typischerweise dazu ausgebildet, einen Skalierungsfaktor für jedes Kernsegment 51, 52, 53, 54, 55 unter Berücksichtigung des Peripheriesignals und einer mittleren Signalintensität der MR-Daten für das jeweilige Kernsegment 51, 52, 53, 54, 55 zu bestimmen. Die Skalierungseinheit 5 ist typischerweise dazu ausgebildet, MR-Daten des Kernbereiches 50 unter Berücksichtigung der MR-Daten jedes Kernsegmentes 51, 52, 53, 54, 55 und dem des jeweiligen Kernsegmentes 51, 52, 53, 54, 55 entsprechenden Skalierungsfaktors zu skalieren. Die Filterungseinheit 6 ist typischerweise dazu ausgebildet, gefilterte MR-Daten durch Kombination der skalierten MR-Daten des Kernbereiches 50 mit den MR-Daten des Peripheriebereiches 42 zu erzeugen und/oder die gefilterten MR-Daten zu Bilddaten zu rekonstruieren. Die Bilddaten können über den Ausgang 7 bereitgestellt werden. Die erfindungsgemäße Rekonstruktionseinheit 1 ist demnach zu einer Ausführung eines erfindungsgemäßen Verfahrens zu einer adaptiven Rekonstruktion von MR-Daten ausgelegt.

Hierzu weist die Rekonstruktionseinheit 1 Computerprogramme und/oder Software auf, die direkt in einer nicht näher dargestellten Speichereinheit der Rekonstruktionseinheit 1 ladbar sind, mit Programmmitteln, um ein Verfahren zu einer adaptiven Rekonstruktion von MR-Daten auszuführen, wenn die Computerprogramme und/oder Software in der Rekonstruktionseinheit 1 ausgeführt werden. Die Rekonstruktionseinheit 1 weist hierzu einen nicht näher dargestellten Prozessor auf, der zu einer Ausführung der Computerprogramme und/oder Software ausgelegt ist. Alternativ hierzu können die Computerprogramme und/oder Software auch auf einem getrennt von der Rekonstruktionseinheit 1 ausgebildeten elektronisch lesbaren Datenträger 21 gespeichert sein, wobei ein Datenzugriff von der Rekonstruktionseinheit 1 auf den elektronisch lesbaren Datenträger 21 über ein Datennetz erfolgen kann.

Ein Verfahren zu einer adaptiven Rekonstruktion von MR-Daten kann auch in Form eines Computerprogrammprodukts vorliegen, das das Verfahren auf die Rekonstruktionseinheit 1 implementiert, wenn es auf der Rekonstruktionseinheit 1 ausgeführt wird. Ebenso kann ein elektronisch lesbarer Datenträger 21 mit darauf gespeicherten elektronisch lesbaren Steuerinformationen vorliegen, welche zumindest ein solches eben beschriebenes Computerprogrammprodukt umfassen und derart ausgestaltet sind, dass sie bei Verwendung des elektronisch lesbaren Datenträgers 21 in einer Rekonstruktionseinheit 1 das beschriebene Verfahren durchführen.

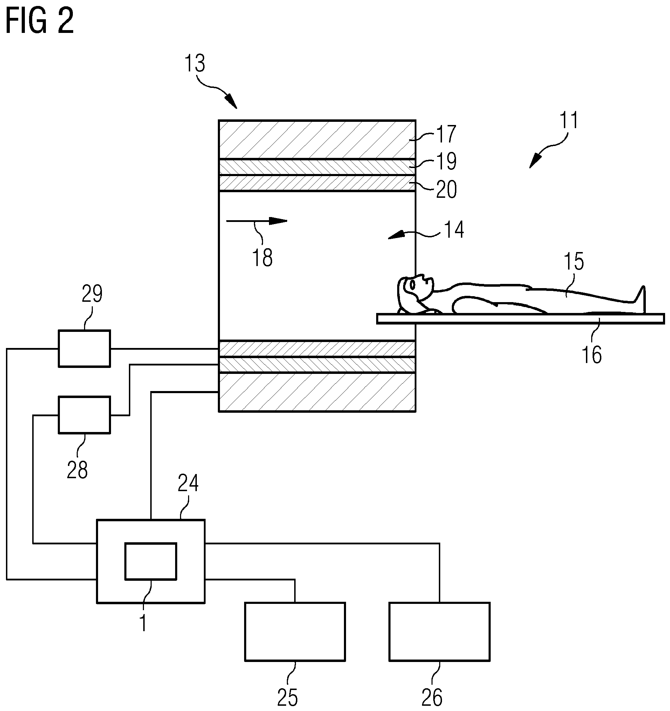

Figur 2 zeigt ein Magnetresonanzgerät 11 zur Ausführung eines erfindungsgemäßen Verfahrens und zur Aufnahme von MR-Daten, umfassend MR-Daten eines Kernbereiches 50 eines k-Raumes 40 und MR-Daten eines Peripheriebereiches 42 des k-Raumes 40 in einer schematischen Darstellung. Das Magnetresonanzgerät 11 umfasst eine von einer Magneteinheit 13 gebildeten Detektoreinheit mit einem Hauptmagneten 17 zu einem Erzeugen eines starken und insbesondere konstanten Hauptmagnetfelds 18. Zudem weist das Magnetresonanzgerät 11 einen zylinderförmigen Patientenaufnahmebereich 14 zu einer Aufnahme eines Patienten 15 auf, wobei der Patientenaufnahmebereich 14 in einer Umfangsrichtung von der Magneteinheit 13 zylinderförmig umschlossen ist. Der Patient 15 kann mittels einer Patientenlagerungsvorrichtung 16 des Magnetresonanzgeräts 11 in den Patientenaufnahmebereich 14 geschoben werden. Die Patientenlagerungsvorrichtung 16 weist hierzu einen Patiententisch auf, der bewegbar innerhalb des Magnetresonanzgeräts 11 angeordnet ist.

Die Magneteinheit 13 weist weiterhin eine Gradientenspuleneinheit 19 auf, die für eine Ortskodierung während einer Bildgebung verwendet wird. Die Gradientenspuleneinheit 19 wird mittels einer Gradientensteuereinheit 28 angesteuert. Des Weiteren weist die Magneteinheit 13 eine Hochfrequenzantenneneinheit 20, welche im gezeigten Fall als fest in das Magnetresonanzgerät 11 integrierte Körperspule ausgebildet ist, und eine Hochfrequenzantennensteuereinheit 29 zu einer Anregung einer Polarisation, die sich in dem von dem Hauptmagneten 17 erzeugten Hauptmagnetfeld 18 einstellt, auf. Die Hochfrequenzantenneneinheit 20 wird von der Hochfrequenzantennensteuereinheit 29 angesteuert und strahlt hochfrequente Hochfrequenz-Pulse in einen Untersuchungsraum, der im Wesentlichen von dem Patientenaufnahmebereich 14 gebildet ist, ein.

Zu einer Steuerung des Hauptmagneten 17, der Gradientensteuereinheit 28 und der Hochfrequenzantennensteuereinheit 29 weist das Magnetresonanzgerät 11 eine Steuerungseinheit 24 auf. Die Steuerungseinheit 24 steuert zentral das Magnetresonanzgerät 11, wie beispielsweise das Durchführen von MR-Steuerungssequenzen. Die Steuerungseinheit 24 ist typischerweise zu einer Ansteuerung des Magnetresonanzgerät 11 für eine Akquisition von MR-Daten des Kernbereiches 50 ausgebildet, wobei ein mehrfaches zeitsequentielles Durchführen der folgenden Schritte, um jeweils die MR-Daten eines Echozuges zu erfassen, erfolgt:
- Schalten eines Anregungspulses 38,
- Schalten von mehreren Refokussierungspulsen 39, wobei zwischen zwei aufeinanderfolgenden Refokussierungspulsen 39 je ein Auslesen einer k-Raum-Zeile 41, insbesondere einem Echo des Echozuges, zugeordnet einem der zumindest drei Kernsegmente 51, 52, 53, 54, 55 erfolgt.

Zudem umfasst die Steuerungseinheit 24 eine erfindungsgemäße Rekonstruktionseinheit 1 zu einer Rekonstruktion von medizinischen Bilddaten, die während der Magnetresonanzuntersuchung erfasst werden. Das Magnetresonanzgerät 11 weist eine Anzeigeeinheit 25 auf. Steuerinformationen wie beispielsweise Steuerungsparameter, sowie rekonstruierte Bilddaten können auf der Anzeigeeinheit 25, beispielsweise auf zumindest einem Monitor, für einen Benutzer angezeigt werden. Zudem weist das Magnetresonanzgerät 11 eine Eingabeeinheit 26 auf, mittels derer Informationen und/oder Steuerungsparameter während eines Messvorgangs von einem Benutzer eingegeben werden können. Die Steuerungseinheit 24 kann die Gradientensteuereinheit 28 und/oder Hochfrequenzantennensteuereinheit 29 und/oder die Anzeigeeinheit 25 und/oder die Eingabeeinheit 26 umfassen.

Das dargestellte Magnetresonanzgerät 11 kann selbstverständlich weitere Komponenten umfassen, die Magnetresonanzgeräte 11 gewöhnlich aufweisen. Eine allgemeine Funktionsweise eines Magnetresonanzgeräts 11 ist zudem dem Fachmann bekannt, so dass auf eine detaillierte Beschreibung der weiteren Komponenten verzichtet wird. Das Magnetresonanzgerät 11 ist somit zusammen mit der Rekonstruktionseinheit 1 zur Ausführung eines erfindungsgemäßen Verfahrens ausgelegt.

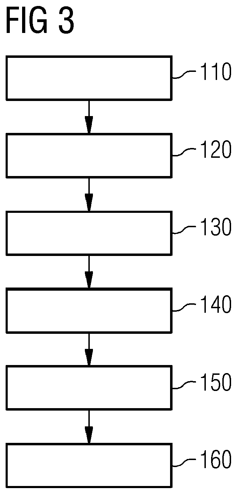

Figur 3 zeigt ein Ablaufdiagramm einer ersten Ausführungsform eines erfindungsgemäßen Verfahrens. Verfahrensschritt 110 sieht die Bereitstellung von MR-Daten umfassend MR-Daten eines Kernbereiches 50 eines k-Raumes 40 und MR-Daten eines Peripheriebereiches 42 des k-Raumes 40 vor. Die MR-Daten des Peripheriebereiches 42 umfassen simuliertes Signal ungleich Null. Die MR-Daten des Kernbereiches 50 sind akquirierte Daten und in zumindest drei Kernsegmente 51, 52, 53, 54, 55 untergliedert. Verfahrensschritt 120 umfasst eine Ermittlung eines Peripheriesignals basierend auf den MR-Daten des Peripheriebereiches 42. Verfahrensschritt 130 umfasst eine Bestimmung eines Skalierungsfaktors für jedes Kernsegment 51, 52, 53, 54, 55 unter Berücksichtigung des Peripheriesignals und einer mittleren Signalintensität der MR-Daten für das jeweilige Kernsegment 51, 52, 53, 54, 55. In Verfahrensschritt 140 erfolgt die Skalierung der MR-Daten des Kernbereiches 50 unter Berücksichtigung der MR-Daten jedes Kernsegmentes 51, 52, 53, 54, 55 und dem des jeweiligen Kernsegmentes 51, 52, 53, 54, 55 entsprechenden Skalierungsfaktors. Verfahrensschritt 150 sieht die Erzeugung gefilterter MR-Daten 61 durch Kombination der skalierten MR-Daten des Kernbereiches 50 mit den MR-Daten des Peripheriebereiches 42 vor. Die gefilterten MR-Daten 61 werden in Verfahrensschritt 160 zu Bilddaten rekonstruiert.

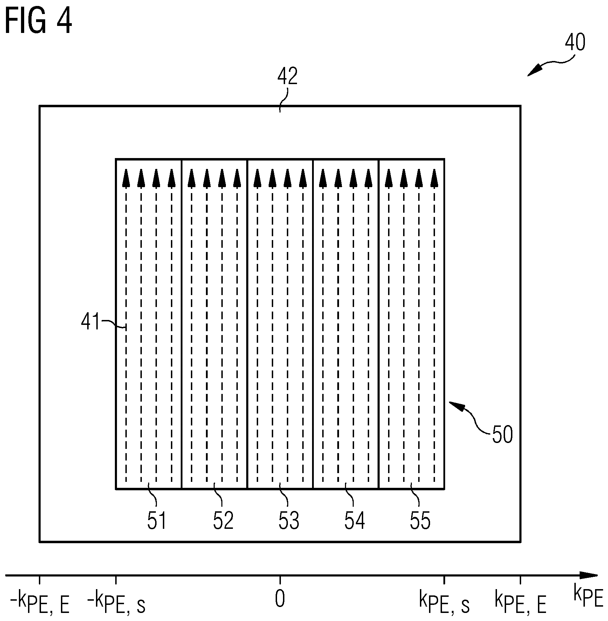

Figur 4 zeigt einen k-Raum 40 in einer schematischen Darstellung. Der k-Raum 40 umfasst einen Peripheriebereich 42, welcher MR-Daten in Form eines simulierten, insbesondere mittels eines neuronalen Netzes simulierten, Signals ungleich Null umfasst. Der k-Raum 40 umfasst einen Kernbereich 50, der in zumindest drei, im dargestellten Fall fünf Kernsegmente 51, 52, 53, 54, 55 untergliedert ist. Jedes Kernsegment 51, 52, 53, 54, 55 umfasst zueinander parallele k-Raum-Zeilen 41 in Richtung der Frequenzkodierung k_{RO}, welche senkrecht zur Phasenkodierrichtung k_{PE} ist. Die Kernsegmente 51, 52, 53, 54, 55 sind derart zueinander angeordnet, dass die von ihnen umfassten parallelen k-Raum-Zeilen 41 zueinander parallel sind. Die Kernsegmente 51, 52, 53, 54, 55 sind typischerweise disjunkt und/oder in Phasenkodierrichtung k_{PE} sequentiell angeordnet. Das Zentrum des k-Raumes 40 entspricht typischerweise dem Zentrum des Kernbereiches 50. Das Zentrum des k-Raumes 40 wird typischerweise vom Kernbereich 50 umfasst.

Der Peripheriebereich 42 kann den Kernbereich 50 vollständig umgeben. Der Kernbereich 50 wird in Phasenkodierrichtung k_{PE} typischerweise durch [-k_{PE,s}, k_{PE,s}] begrenzt.

Der Peripheriebereich 42 schließt typischerweise an den peripheren Rändern des Kernbereiches 50 in Phasenkodierrichtung k_{PE} an. Der Peripheriebereich 42 umfasst typischerweise vollständige k-Raum-Zeilen 41 nur außerhalb des Teilbereiches [-k_{PE,s}, k_{PE,s}] in Phasenkodierrichtung k_{PE}. Innerhalb des Teilbereiches [-k_{PE,s}, k_{PE,s}] liegende k-Raum-Zeilen 41 werden höchstens teilweise vom Peripheriebereich 42 umfasst, insbesondere nur für hohe Werte für k in Richtung der Frequenzkodierung k_{RO}. Der Peripheriebereich 42 kann durch den Wertebereich [-k_{PE,E}, -k_{PE,s}[∪] k_{PE,s}, k_{PE,E}] oder durch [-∞, -k_{PE,s} [∪] k_{PE,s}, ∞] in Phasenkodierrichtung k_{PE} beschrieben werden. Der Peripheriebereich 42 ist frei vom Zentrum des k-Raumes 40. Das Zentrum des k-Raumes 40 ist definiert durch k_{PE} = 0, k_{RO} = 0.

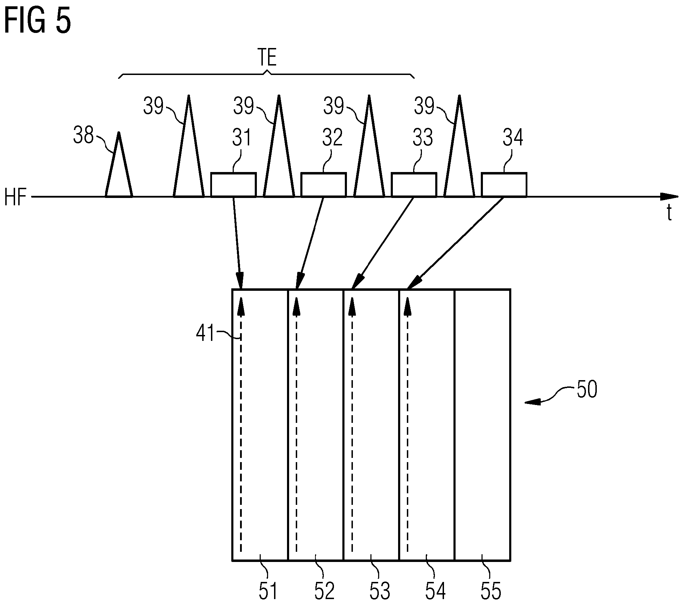

Figur 5 zeigt ein Erfassen von MR-Daten des Kernbereiches 50 in einem ersten Echozug in einer schematischen Darstellung. Der erste Echozug ist durch die MR-Steuerungssequenz definiert und umfasst einen Anregungspuls 38 gefolgt von mehreren Refokussierungspulsen 39, wobei zwischen zwei aufeinanderfolgenden Refokussierungspulsen 39 je ein Auslesen einer k-Raum-Zeile 41, insbesondere bei jedem Echo 31, 32, 33, 34 des Echozuges, erfolgt. Es ist in Figur 5 die Amplitude der HF-Pulse, und die resultierenden Echos in Abhängigkeit der Zeitachse dargestellt. Die MR-Daten des ersten Echos 31 werden einer k-Raum-Zeile 41 des ersten Kernsegmentes 51 zugeordnet. Die MR-Daten des zweiten Echos 32 werden einer k-Raum-Zeile 41 des zweiten Kernsegmentes 52 zugeordnet. Die MR-Daten des dritten Echos 33 werden einer k-Raum-Zeile 41 des dritten Kernsegmentes 53 zugeordnet. Die MR-Daten des vierten Echos 34 werden einer k-Raum-Zeile 41 des vierten Kernsegmentes 54 zugeordnet.

Dieser Echozug wird vorzugsweise derart häufig wiederholt, bis die k-Raum-Zeilen 41 der Kernsegmente 51, 52, 53, 54 mit MR-Daten befüllt sind, was insbesondere von der Größe der Kernsegmente 51, 52, 53, 54 und/oder der Anzahl der k-Raum-Zeilen 41 pro Kernsegment 51, 52, 53, 54 abhängig ist. Dabei ist der zeitliche Abstand dt des Auslesens aller einem Kernsegment der Kernsegmente 51, 52, 53, 54 zugeordneten k-Raum-Zeilen 41 zum Anregungspuls 38 des entsprechenden Echozuges vorzugsweise gleich. Im dargestellten Fall umfasst das dritte Kernsegment 53 das Zentrum des k-Raumes 40 und es wird durch die MR-Daten des dritten Echos 33 befüllt. Der zeitliche Abstand dt des dritten Echos 33 vom Anregungspuls 38 definiert demnach typischerweise die Echozeit T_{E} der MR-Steuerungssequenz.

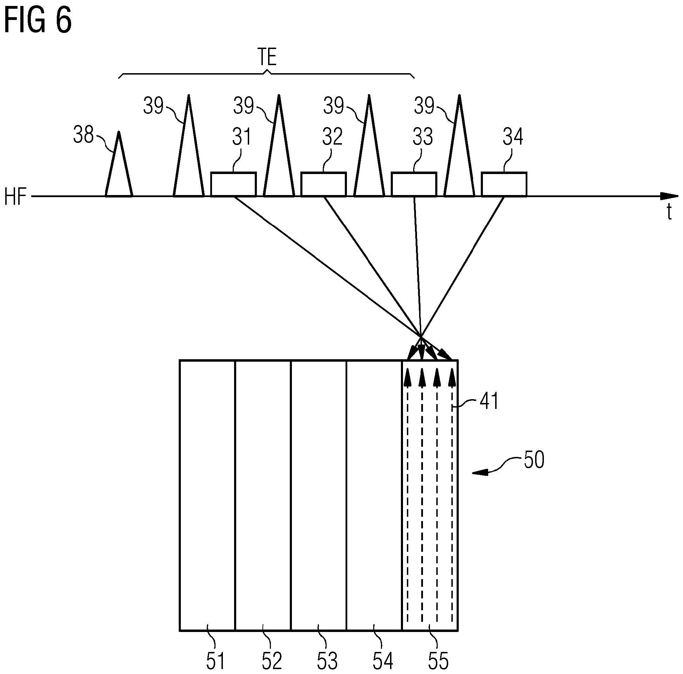

Figur 6 zeigt ein Erfassen von MR-Daten des Kernbereiches 50 in einem weiteren Echozug in einer schematischen Darstellung.

Figur 6 unterscheidet sich von Figur 5 darin, dass MR-Daten aller Echos 31, 32, 33, 34 des weiteren Echozuges zur Füllung der k-Raum-Zeilen 41 im fünften Kernsegment 55 verwendet werden. Der zeitliche Abstand dt des Auslesens der dem fünften Kernsegment 55 zugeordneten k-Raum-Zeilen 41 zum Anregungspuls 38 des entsprechenden Echozuges ist voneinander verschieden.

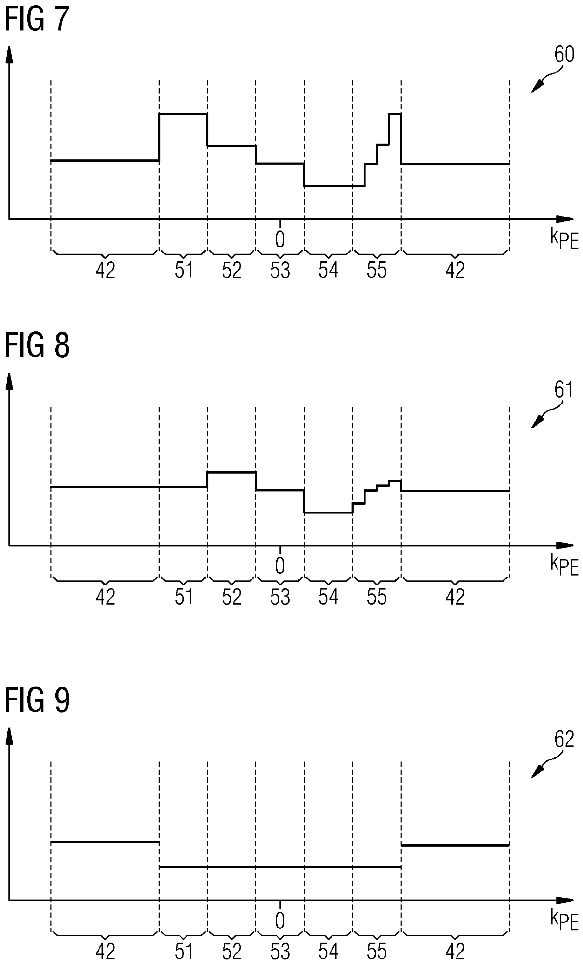

Figur 7 zeigt eine Signalintensität 60 der MR-Daten vor der Skalierung in Abhängigkeit von der Position in Phasenkodierrichtung k_{PE}. Diese Signalintensität 60 der MR-Daten vor der Skalierung kann beispielsweise durch ein Erfassen von MR-Daten des Kernbereiches 50 gemäß dem in den Figuren 5 und 6 beschriebenen Verfahren erzielt werden. Hierbei ist zu beachten, dass die Signalintensität 60 der erfassten MR-Daten typischerweise proportional zu exp(- dt / T₂) ist. T₂ ist typischerweise eine mittlere T₂ -Relaxationszeit zumindest eines Teilbereiches des Untersuchungsbereiches. Die Signalintensität 60 der erfassten MR-Daten ist demnach typischerweise vom zeitlichen Abstand dt des Auslesens der jeweiligen k-Raum-Zeile 41 zum Anregungspuls 38 abhängig. Die Bestimmung des Skalierungsfaktors erfolgt vorzugsweise unter Berücksichtigung des zeitlichen Abstandes dt des Auslesens einer dem jeweiligen Kernsegment 51, 52, 53, 54, 55 zugeordneten k-Raum-Zeile 41 zum Anregungspuls 38.

Figur 8 zeigt eine erste Signalintensität 61 von gefilterten MR-Daten mit einem ersten Skalierungsfaktor gemäß einer ersten Ausführungsform.

Der erste Skalierungsfaktor ist vorzugsweise proportional zu exp(- T₂ / dt) und/oder durch A* exp(- T₂ / dt) + B beschreibbar, wobei und A und B Optimierungsparameter im Rahmen der Bestimmung des Skalierungsfaktors sind. Verfahrensschritt 130, also die Bestimmung des Skalierungsfaktors, kann derart erfolgen, dass die Differenz zwischen dem Peripheriesignal und der mittleren Signalintensität der skalierten MR-Daten eines dem Peripheriebereich 42 benachbarten Kernsegments 51, 55 minimiert wird. Dies kann durch Optimierung von A und B unter Berücksichtigung von A* exp(- T₂ / dt) + B für den ersten Skalierungsfaktor erzielt werden.

Figur 9 zeigt eine zweite Signalintensität 62 von gefilterten MR-Daten mit einem Skalierungsfaktor gemäß einer zweiten Ausführungsform. Der zweite Skalierungsfaktor ist vorzugsweise durch A* exp(- T₂ / dt) + B beschreibbar,
wobei A und B derart optimiert werden, dass dem dritten Kernsegment 53, also dem Kernsegment umfassend MR-Daten mit dem zeitlichen Abstand dt entsprechend einer definierten Echozeit T_{E}, der Skalierungsfaktor gleich eins zugewiesen wird.

Obwohl die Erfindung im Detail durch die bevorzugten Ausführungsbeispiele näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zu einer adaptiven Rekonstruktion von MR-Daten umfassend die folgenden Verfahrensschritte:
- Bereitstellung von MR-Daten umfassend MR-Daten eines Kernbereiches eines k-Raumes und MR-Daten eines Peripheriebereiches des k-Raumes, wobei
- der Kernbereich des k-Raumes zumindest drei Kernsegmente umfasst,
- jedes Kernsegment der zumindest drei Kernsegmente zueinander parallele k-Raum-Zeilen umfasst und die Kernsegmente derart zueinander angeordnet sind, dass die von ihnen umfassten parallelen k-Raum-Zeilen zueinander parallel sind,
- die MR-Daten des Peripheriebereiches simuliertes Signal ungleich Null umfassen,
- die MR-Daten des Kernbereiches durch eine Akquisition umfassend ein mehrfaches zeitsequentielles Durchführen der folgenden Schritte, um jeweils die MR-Daten eines Echozuges zu erfassen, gekennzeichnet sind:
- Schalten eines Anregungspulses,
- Schalten von mehreren Refokussierungspulsen, wobei zwischen zwei aufeinanderfolgenden Refokussierungspulsen je ein Auslesen einer k-Raum-Zeile, insbesondere einem Echo des Echozuges, zugeordnet einem der zumindest drei Kernsegmente erfolgt,
- Ermittlung eines Peripheriesignals basierend auf den MR-Daten des Peripheriebereiches,
- Bestimmung eines Skalierungsfaktors für jedes Kernsegment unter Berücksichtigung des Peripheriesignals und einer mittleren Signalintensität der MR-Daten für das jeweilige Kernsegment,
- Skalierung der MR-Daten des Kernbereiches unter Berücksichtigung der MR-Daten jedes Kernsegmentes und dem des jeweiligen Kernsegmentes entsprechenden Skalierungsfaktors,
- Erzeugung gefilterter MR-Daten durch Kombination der skalierten MR-Daten des Kernbereiches mit den MR-Daten des Peripheriebereiches,
- Rekonstruktion von Bilddaten aus den gefilterten MR-Daten.

2. Verfahren nach Anspruch 1,
wobei die Bestimmung des Skalierungsfaktors derart erfolgt, dass die Differenz zwischen dem Peripheriesignal und der mittleren Signalintensität der MR-Daten eines dem Peripheriebereiches benachbarten Kernsegment minimiert wird.

3. Verfahren nach einem der vorangehenden Ansprüche,
wobei die Bestimmung des Skalierungsfaktors unter Berücksichtigung eines zeitlichen Abstandes dt des Auslesens einer dem jeweiligen Kernsegment zugeordneten k-Raum-Zeile zum Anregungspuls erfolgt.

4. Verfahren nach Anspruch 3,
wobei der zeitliche Abstand dt den Skalierungsfaktor S gemäß der Abhängigkeit S ∼ exp(- T₂ / dt) beeinflusst, wobei T₂ eine mittlere T₂-Relaxationszeit zumindest eines Teilbereiches des Untersuchungsbereiches ist.

5. Verfahren nach einem der Ansprüche 3 bis 4,
wobei der zeitliche Abstand dt den Skalierungsfaktor S gemäß der Abhängigkeit S = A* exp(- T₂ / dt) + B beeinflusst, wobei T₂ eine mittlere T₂-Relaxationszeit zumindest eines Teilbereiches des Untersuchungsbereiches ist und A und B Optimierungsparameter im Rahmen der Bestimmung des Skalierungsfaktors sind.

6. Verfahren nach Anspruch 5,
wobei A und B derart optimiert werden, dass die Differenz zwischen dem Peripheriesignal und der mittleren Signalintensität der MR-Daten von zwei an den Peripheriebereich direkt angrenzenden Kernsegmenten der zumindest drei Kernsegmente minimiert wird.

7. Verfahren nach Anspruch 5,
wobei A und B derart optimiert werden, dass dem Kernsegment umfassend MR-Daten mit dem zeitlichen Abstand dt entsprechend einer definierten Echozeit der Skalierungsfaktor gleich eins zugewiesen wird.

8. Verfahren nach einem der vorangehenden Ansprüche,
wobei die MR-Daten des Peripheriebereiches mittels eines neuronalen Netzes simuliertes Signal umfassen.

9. Verfahren nach einem der vorangehenden Ansprüche,
wobei der zeitliche Abstand dt des Auslesens aller einem Kernsegment der zumindest drei Kernsegmenten zugeordneten k-Raum-Zeilen zum Anregungspuls des entsprechenden Echozuges gleich ist.

10. Verfahren nach einem der vorangehenden Ansprüche,
wobei der zeitliche Abstand dt des Auslesens von zumindest einem Kernsegment der zumindest drei Kernsegmenten zugeordneten k-Raum-Zeilen zum Anregungspuls des entsprechenden Echozuges voneinander verschieden ist.

11. Rekonstruktionseinheit umfassend eine Ermittlungseinheit und eine Skalierungseinheit, die zu einer Ausführung eines Verfahrens zu einer adaptiven Rekonstruktion von MR-Daten nach einem der vorangehenden Ansprüche ausgelegt ist.

12. Magnetresonanzgerät mit einer Steuerungseinheit umfassend eine Rekonstruktionseinheit, die zu einer Ausführung eines Verfahrens zu einer adaptiven Rekonstruktion von MR-Daten nach einem der Ansprüche 1 bis 10 ausgelegt ist.

13. Computerprogrammprodukt, welches ein Programm umfasst und direkt in einen Speicher einer programmierbaren Rekonstruktionseinheit ladbar ist, mit Programmmitteln, um ein Verfahren zu einer adaptiven Rekonstruktion von MR-Daten nach einem der Ansprüche 1 bis 10 auszuführen, wenn das Programm in der Rekonstruktionseinheit ausgeführt wird.
